# EUROPEAN PATENT APPLICATION

(11) **EP 4 710 932 A1**
(43) Date of publication of application: **18.03.2026**
(21) Application number: 24382973.6
(22) Date of filing: 12.09.2024
(51) Int. Cl.: A61K 33/32, A61K 9/00, A61P 25/00, A61P 25/14, A61P 25/16, A61P 25/28, G01N 33/50

(54) **COMPOSITIONS COMPRISING MANGANESE FOR USE IN THE TREATMENT OF A NEURODEGENERATIVE DISEASE**

(71) Applicant: Administración General de la Comunidad Autónoma de Euskadi, 01010 Vitoria-Gasteiz, Álava (ES)
(72) Inventor: HOLT, Ian, San Sebastián, Gipuzkoa (ES); LÓPEZ DE ARBINA, Amaia, San Sebastián, Gipuzkoa (ES); SPINAZZOLA, Antonella, London, WC1E 6BT (GB)
(74) Representative: Pons IP

(57) **Abstract**

The invention relates to composition comprising manganese, a solvate or a pharmaceutically acceptable salt thereof, for use in the treatment of a disease in a subject, wherein the subject's cells have fewer than half the number of functional endoplasmic reticulum and mitochondria contact sites than the cells from a subject not suffering from the disease, and to an in vitro method for designing a personalised therapy to a subject suffering from a disease, comprising determining the mtDNA synthesis rate in a biological sample isolate from the subject in the presence and absence of Mn.

## Description

The invention relates to a composition comprising manganese for use in the treatment of diseases linked to aberrant endoplasmic reticulum and mitochondria contact sites (ERMCS), to an *in vitro* method for designing a personalised therapy to a subject suffering from a disease linked to aberrant ERMCs. Thus, the present invention belongs to the medical field, specifically, to the treatment of disorders where the ERMCS are non-functional, including but not limited to disease of the brain and nervous system.

### BACKGROUND ART

Neurological and neurodegenerative disorders (NNDs) affect millions of people worldwide. Although age is the single most contributing risk factor to the development of NNDs, recent findings reveal that a combination of an individual's genetic makeup and environmental factors can contribute to increasing the risk for NNDs. Further, despite the expression of specific genes (within an individual) accountable for NNDs, the time and extent of neurodegeneration depend on the environment. More recent studies reveal that multiple pathologies may underline a single neurodegenerative disorder.

Since the brain controls several aspects of the body's function, neurodegenerative diseases consequently affect multiple facets of human functioning and limit the ability to perform both basic (e.g., speech, movement, stability, and balance) and complicated tasks (e.g., bladder and bowel functions, and cognitive abilities). Most NNDs progress without remission, whilst in some cases, treatments target the improvement of symptoms, relief of pain if present and/or the restoration of balance and mobility.

Energy production by mitochondria depends on 13 proteins encoded by the small circles of DNA located inside the organelles. The mitochondrial DNA (mtDNA) molecules form nucleoprotein complexes, or nucleoids, that are closely and tightly associated with the inner of the two mitochondrial membranes. It has been proposed that the mtDNA is supported in the inner mitochondrial membrane by cholesterol micro-domains, as the small amount of cholesterol associated with mitochondria chiefly co-fractionates with the mtDNA after mitochondrial lysis with mild detergent. Moreover, cholesterol appears to be functionally important for mtDNA metabolism, as pharmacological or genetic perturbation of cholesterol homeostasis induces mtDNA abnormalities. Likewise, cholesterol's yeast counterpart, ergosterol, is essential for mtDNA maintenance.

Cholesterol-rich micro-domains are also integral to the contact sites formed between the Endoplasmic Reticulum (ER) and the mitochondria (ERMCS). ERMCS facilitate mitochondrial division and impact mtDNA replication and segregation. However, little is known about the ERMCS proteins that contribute to mtDNA maintenance, nor how the ER regulates mtDNA replication, and how it can be involved in the development of diseases.

Thus, there is in the state of the art the necessity to provide new treatments and diagnosing methods of diseases linked to aberrant ERMCS.

### DESCRIPTION OF THE INVENTION

A growing body of work demonstrates that intracellular connections between the Endoplasmic Reticulum and mitochondria play multiple important roles in regulating mitochondrial activities, including the replication of the small multitudinous DNAs in the mitochondria. The mtDNA is essential for energy production and defects in these molecules cause a range of human diseases, which with time almost invariably lead to neurological disease. There are also a growing number of studies that indicate that the connections between the ER and the mitochondria are often disrupted in neurological and neurodegenerative disorders.

The inventors have discovered that ERMCS support mtDNA replication via the protein ERLIN2 (ER lipid-raft associated 2), which is a protein component of ER lipid micro-domains, with cholesterol-binding properties that serves as a canonical marker of ERMCS. Further inventor's investigations have revealed that:
i) repressing the expression of ERLIN2 or other components of ERMCS, specifically RTN4 and GRP75, inhibits mitochondrial DNA replication;
ii) the expression of a carrier protein (called the mitochondrial calcium uniporter, or MCU) in the mitochondrial inner membrane that regulates the entry of calcium and manganese into the mitochondrial is repressed when some ERMCS factors are scarce (i.e. after RTN4 or GRP75 silencing); and a chemical inhibitor of MCU* inhibits mtDNA replication similar to RTN4 or GRP75 silencing (*the inhibitor is called MCU-i4 that is a negative modulator of mitochondrial calcium (Ca 2+) uniporter (MCU) complex, which binds to MICU1, a regulatory protein within the MCU complex);
iii) silencing superoxide dismutase 2 (SOD2, aka manganese-dependent superoxide dismutase) inhibits mtDNA replication;
iv) mitochondrial DNA synthesis can be restored in contexts i-iii above (ERMCS silencing, the MCU inhibitor, or SOD2 silencing) by supplementing cells with the metal cation manganese;

In view of the above results, the inventors realized that the abundance of functional ERMCS is inversely proportional to the increase in mtDNA synthesis elicited by manganese supplementation. Thus, mtDNA synthesis is altered little or not at all in samples from healthy control subjects, whereas in the case of ERMCS dysfunction or scarcity, manganese will produce a 2-fold or greater increase in mtDNA synthesis rate, which is indicative that the manganese is useful in the treatment of a disease in a subject whose cells have too few functional ERMCS to support mtDNA replication with respect to the cells from a subject not suffering from the disease.

Therefore, in one aspect, the present invention relates to a composition comprising manganese (Mn²⁺), a solvate or a pharmaceutically acceptable salt thereof, hereinafter "composition of the invention", for use in the treatment of a disease in a subject, wherein the subject's cells have fewer than half the number of functional endoplasmic reticulum and mitochondria contact sites (ERMCS), or MCU or SOD2, than the cells from a subject not suffering from the disease. (hereinafter "use of the invention"). Most human diseases are recessive (including several involving factors implicated in ER-mitochondrial interactions and mtDNA metabolism - Mitofusin2 and paraplegin as examples) because 50% (half) of the normal amount of a protein or complex is usually sufficient to avoid disease. Moreover, mtDNA diseases manifest when there is less than half the normal number of functional molecules. Therefore, decreases in mtDNA synthesis rate of greater than 50% are predicted to be indicative of a disease for which manganese could be beneficial. Notwithstanding this, not all individuals displaying decreases in mtDNA synthesis of 50% or greater will benefit from manganese, and those that can benefit from manganese can be determined by applying a mtDNA synthesis assay with and without manganese in peripheral cells and tissues (see below), as only those that can benefit from manganese will display an increase in mtDNA synthesis rate to above the 50% normal control rate in the presence of manganese compared to in its absence.

As use herein, manganese is a chemical element with the symbol Mn and atomic number 25. Common oxidation states of manganese include, without limiting to, +2, +3, +4, +6, and +7. From the biological point of view, manganese is an essential human dietary element. It is present as a coenzyme in several biological processes, which include macronutrient metabolism, bone formation, and free radical defence systems.

The present invention encompasses a solvate or a pharmaceutically acceptable salt of manganese. The term "solvate" used herein refers to a crystalline compound in which molecules of solvents are incorporated into the crystal lattice of the compound. When the solvent is water, the solvate is an "hydrate", which refers to a crystalline compound in which molecules of water are incorporated into the crystal lattice of the compound. Examples of hydrates within the scope of the present inventions are manganese sulfate monohydrate (zmikite); tetrahydrate (ilesite); pentahydrate (jo̅kokuite); hexahydrate (chvaleticeite); heptahydrate (mallardite). Manganese chloride monohydrate, dihydrate, tetrahydrate (Lumenhance), hexahydrate. Manganese dioxide hydrate; manganese acetate tetrahydrate; manganese gluconate dihydrate. The term "pharmaceutically acceptable salt" refers to a salt that is appropriate for use in clinical or pharmaceutical contexts, i.e., salt which is suitable for administration in animals or humans without undue toxicity, irritation, allergic response and the like, and are commensurate with a reasonable benefit/risk ratio. Examples of pharmaceutically acceptable salts include, without limiting to:
- Manganese chloride that is used as a dietary supplement/food additive, and in animal feed;
- Manganese sulfate that is used in medicines and as a nutritional supplement;
- Manganese gluconate that is used as a feed additive, food additive and dietary supplement;
- Manganese dioxide that is used as food additive and a dietary supplement;
- Manganese acetate that is used in feed additives, and in manufacturing;
- Manganese carbonate that is used in medications, and as a plant nutrient.

Any subject suffering from a disease and characterized by having cells with less than 50% of functional ERMCS in comparison with, the cells from a subject not suffering from the disease, can be treated with Mn²⁺.

Examples of diseases which may be treated in the context of the present invention include, but not limited to Alzheimer's disease (including mild or early-stage Alzheimer's disease, mild to moderate Alzheimer's disease, moderate or mid-stage Alzheimer's disease, moderate to severe Alzheimer's disease, moderately severe Alzheimer's disease, severe Alzheimer's disease, Alzheimer's disease with Lewy bodies, (AD), Parkinson's disease (including Parkinson's disease chemically induced by exposure to environmental agents such as pesticides, insecticides, or herbicides and/or metals such as aluminium, cadmium, copper, or zinc, SNCA gene-linked Parkinson's disease, sporadic or idiopathic Parkinson's disease, or Parkin- or LRRK2-linked Parkinson's disease (PD), autosomal-dominant Parkinson's disease, Diffuse Lewy Body Disease (DLBD) also known as Dementia with Lewy Bodies (DLB), Pure Autonomic Failure, Lewy body dysphagia, Incidental LBD, Inherited LBD (e.g., mutations of the alpha-synuclein gene, PARK3 and PARK4), multiple system atrophy (including Olivopontocerebellar Atrophy, Striatonigral Degeneration, Shy-Drager Syndrome (MSA), combined Alzheimer's and Parkinson disease and/or MSA, Huntington's disease, synucleinopathies, disorders or conditions characterized by the presence of Lewy bodies, multiple sclerosis, Amyotrophic lateral sclerosis (ALS) dementia (including vascular dementia, Lewy body dementia, Parkinson's dementia, frontotemporal dementia), Frontal Temporal Dementia (FTD), Cerebellar Ataxia, Peripheral Neuropathy, Hereditary Spastic Paraplegia, Charcot-Marie Tooth, Motor Neuron Disease, Mitochondrial Disorders, Down syndrome, Psychosis (including agitation caused by a neurodegenerative disease or associated with dopaminergic therapy such as, but not limited to, Parkinson's disease psychosis, Alzheimer's disease psychosis, Lewy body dementia psychosis), dyskinesia (including agitation caused by a neurodegenerative disease or associated with dopaminergic therapy), agitation (including agitation caused by a neurodegenerative disease or associated with dopaminergic therapy), conditions associated with dopaminergic therapy (including dystonia, myoclonus, or tremor), diseases, disorders or conditions associated with abnormal expression, stability, activities and/or cellular processing of alpha-synuclein, and combinations thereof.

As use herein, ERMCS refers to the Endoplasmic reticulum-mitochondria contact sites (also named ERMCS), which are dynamic contact regions with a distance of 10-30 nm between the endoplasmic reticulum and mitochondria. As it is widely known, the dysfunction, abnormality or impairment in the ERMCS is closely associated with various neurodegenerative diseases, including Parkinson's disease, Alzheimer's disease, and amyotrophic lateral sclerosis, among others. In the context of the present invention, the ERMCS are aberrant, impeded, dysfunctional or abnormal (all these terms may be used as equivalent terms) when the ERMCS are not capable of carrying out their function, i.e. fundamental cellular processes such as Ca²⁺ exchange between the two organelles, uptake of Ca²⁺ by mitochondria following its release from ER stores via inositol 1,4,5-trisphosphate (IP3) receptors; or phospholipid exchange between the ER and the MT; or intracellular trafficking of mitochondria and ER; among others. On the contrary, ERMCS are functional ("functional ERMCS") when they can support the above-mentioned functions.

A method for assessing if the ERMCS, MCU or SOD2 are aberrant, impeded, dysfunctional or abnormal is, for example, mtDNA synthesis assay. The basic measure is the rate of DNA synthesis in the mitochondria, which is determined by incorporating a modified nucleotide, such as bromo-deoxyuridine labelling of mtDNA. This assay will involve taking peripheral tissues, e.g. peripheral blood mononuclear (PBMC) or other blood cells, or buccal cells, or skin fibroblasts cultureed in the laboratory and assaying mtDNA synthesis rates via a nucleotide analogue incorporation assay. Synthesis rates are evaluated in cells of age-matched healthy control subjects, treated with and without manganese versus subjects with diseases potentially attributable to ER-mitochondrial connection or MCU or SOD2 problems. The disease subjects are expected to have a mtDNA synthesis rate no more than 50% of the mean control value, which will be restored to control levels in the presence of manganese. This assay will both confirm there is an ER-mitochondrial connection problem and that the subject is suitable for the manganese-based therapy. This approach could be adapted to an *in vivo* setting as manganese is paramagnetic and can be detected by PET/MRI.

The composition of the invention may comprise, further to the manganese, other therapeutic agents such as antibiotics, painkillers, etc., or other therapeutic agents useful in the treatment of neurodegenerative diseases. Thus, in another particular embodiment of the use of the invention, the composition of the invention comprises other bioactive components or compounds useful in the treatment of neurodegenerative diseases.

Likewise, the composition of the invention may comprise an excipient and/or carrier, preferably, a pharmaceutically acceptable excipient and/or a pharmaceutically acceptable carrier.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. In a particular embodiment, the carrier is pharmaceutically acceptable.

The composition of the present invention can also take the form of a sustained release drug formulation or any other conventional release system, such as, for example, nanoparticles, liposomes or nanospheres, polymeric material, biodegradable or non-biodegradable implant, or biodegradable microparticles, such as, for example, biodegradable microspheres. Biodegradable particles (nanoparticles or microparticles) have been shown to be capable of delivering a variety of therapeutic agents including molecules such as polypeptides or proteins. Thus, in a particular embodiment of the use of the invention, the composition of the invention is comprised within a nanoparticle.

The composition of the invention may be employed as a therapeutic agent to be administered to a subject. In this case, the composition provided by this invention is considered a pharmaceutical composition which may be administered by any appropriate administration route; to this end, said composition will be formulated in the suitable form for the selected administration route.

The "galenic form" or "pharmaceutical form" is the arrangement to which the active ingredients and excipients adapt in order to form a medicament. It is defined by the combination of the form in which the pharmaceutical composition is presented by the manufacturer and the form in which it is administered.

Routes of administration include topical, ocular, nasal, pulmonary, buccal, parenteral (intravenous, subcutaneous, and intramuscular), oral, vaginal and rectal. Administration from implants is also possible. The composition of the invention, in particular the pharmaceutical composition, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms.

As explained at the beginning of the present description, the composition of the invention can be used in the treatment of a disease, in particular, a neurodegenerative disease. The term "neurodegenerative disease" refers to any disease produce by the progressive loss of structure and/or functions of nerve cells in the brain or peripheral nervous system, which eventually involve cell death. Examples of neurodegenerative disease include, without limiting to, Alzheimer's disease (including mild or early-stage Alzheimer's disease, mild to moderate Alzheimer's disease, moderate or mid-stage Alzheimer's disease, moderate to severe Alzheimer's disease, moderately severe Alzheimer's disease, severe Alzheimer's disease, Alzheimer's disease with Lewy bodies, (AD)), Parkinson's disease (including Parkinson's disease chemically induced by exposure to environmental agents such as pesticides, insecticides, or herbicides and/or metals such as aluminium, cadmium, copper, or zinc, SNCA gene-linked Parkinson's disease, sporadic or idiopathic Parkinson's disease, or Parkin- or LRRK2-linked Parkinson's disease (PD)), autosomal-dominant Parkinson's disease, Diffuse Lewy Body Disease (DLBD) also known as Dementia with Lewy Bodies (DLB), Pure Autonomic Failure, Lewy body dysphagia, Incidental LBD, Inherited LBD (e.g., mutations of the alpha-synuclein gene, PARK3 and PARK4), multiple system atrophy (including Olivopontocerebellar Atrophy, Striatonigral Degeneration, Shy-Drager Syndrome (MSA)), combined Alzheimer's and Parkinson disease and/or MSA, Huntington's disease, synucleinopathies, disorders or conditions characterized by the presence of Lewy bodies, multiple sclerosis, Amyotrophic lateral sclerosis (ALS) dementia (including vascular dementia, Lewy body dementia, Parkinson's dementia, frontotemporal dementia), Frontal Temporal Dementia (FTD), Cerebellar Ataxia, Peripheral Neuropathy, Hereditary Spastic Paraplegia, Charcot-Marie Tooth, Motor Neuron Disease, Mitochondrial Disorders, Down syndrome, Psychosis (including agitation caused by a neurodegenerative disease or associated with dopaminergic therapy such as, but not limited to, Parkinson's disease psychosis, Alzheimer's disease psychosis, Lewy body dementia psychosis), dyskinesia (including agitation caused by a neurodegenerative disease or associated with dopaminergic therapy), agitation (including agitation caused by a neurodegenerative disease or associated with dopaminergic therapy), conditions associated with dopaminergic therapy (including dystonia, myoclonus, or tremor), diseases, disorders or conditions associated with abnormal expression, stability, activities and/or cellular processing of alpha-synuclein, and combinations thereof.

Nevertheless, in a particular embodiment of the use of the invention, the disease is a neurodegenerative disease. In another more particular embodiment, the neurodegenerative disease is selected from Alzheimer's disease, Parkinson's disease, Diffuse Lewy Body Disease (DLBD), Huntington's disease, multiple sclerosis, or Amyotrophic lateral sclerosis (ALS) dementia.

In another aspect, the present invention relates to an *in vitro* or *ex vivo* method for designing a therapy to a subject suffering from a disease, hereinafter "method of the invention", comprising
(a) determining the mtDNA synthesis rate in a biological sample isolate from the subject in the presence and absence of Mn, and
(b) comparing the mtDNA synthesis rates obtained in step (a),
wherein
- The mtDNA synthesis rate is measure by a mtDNA synthesis assay, and
- A 2-fold or greater increase in mtDNA synthesis rate in the presence of Mn with respect to the mtDNA synthesis rate in the absence of Mn, is indicative that the therapy to be administered to the subject is a composition comprising manganese, a solvate or a pharmaceutically acceptable salt thereof.

All the terms used in the present inventive aspect have been defined above for the use of the invention, being applicable to the method of the invention.

As used herein, "mtDNA synthesis assay" refers to incorporating a modified nucleotide, such as bromo-deoxyuridine, in mtDNA over a defined time period (typically 1-24 hours). Post-labelling detection depends on the type of analogue, which may be detected by antibodies, click-chemistry or radioactivity as examples. The mtDNA can be detected in situ in cells or after isolation and enrichment by a variety of standard methods.

As used herein, the "mtDNA synthesis rate" refers to the speed or frequency at which mitochondrial DNA (mtDNA) molecules are replicated within a cell, which is influenced by various factors such as the cell's energy demands, the availability of nucleotides, the presence of specific transcription factors, nutrient availability or others. For example, in a particular embodiment of the present method of the invention, the mtDNA synthesis rate is defined as the amount of BrdU incorporated in cytoplasmic foci with an anti-BrdU antibody that coincide with mitochondria (labelled with say anti-TOMM20 antibody) after an 8-hour incubation with BrdU.

In addition, the inventors have observed that the Mn dose to be administered to the subject is proportional to the increase of the mtDNA synthesis rate in the presence of Mn with respect to the mtDNA synthesis rate without Mn. Thus, the method of the invention is useful not only to design a therapy to a subject suffering from a disease, but also to determine the dose of Mn to be administered to the subject. Thus, in a particular embodiment of the method of the invention, the dose of Mn to be administered to the subject will be proportional to the increase of the mtDNA synthesis rate in the presence of Mn with respect to the mtDNA synthesis rate without Mn.

In another particular embodiment of the method of the invention, the disease is a neurodegenerative disease. In a more particular embodiment of the method of the invention, the neurodegenerative disease is selected from Alzheimer's disease, Parkinson's disease, Diffuse Lewy Body Disease (DLBD), Huntington's disease, multiple sclerosis, Amyotrophic lateral sclerosis (ALS), and Fronto temporal dementia (FTD).

In another particular embodiment of the method of the invention, the composition is comprised within a nanoparticle. As used in the present description, the term "nanoparticle" refers to colloidal systems of the spherical, cylindrical, polyhedral, etc. type, or similar shapes, that are less than or equal to 1 µm in size, which can be found individually or forming organised structures (dimer, trimers, etc.), dispersed in a fluid (aqueous solution). In a particular embodiment, nanoparticles suitable for putting the invention into practice have a size smaller than 1 µm, generally between 1 and 99 nanometres (nm), typically between 5 and 500 nm, preferably between about 10 and 150 nm. In a particular embodiment, nanoparticles have a mean diameter between 2 and 50 nm, preferably between 5 and 20 nm. The mean particle diameter is the maximum mean particle dimension, understanding that nanoparticles do not necessarily have to be spherical. Suitable nanoparticles for use in the present invention include polymeric nanoparticles, lipid nanoparticles and metallic nanoparticles.

### Description of the drawings

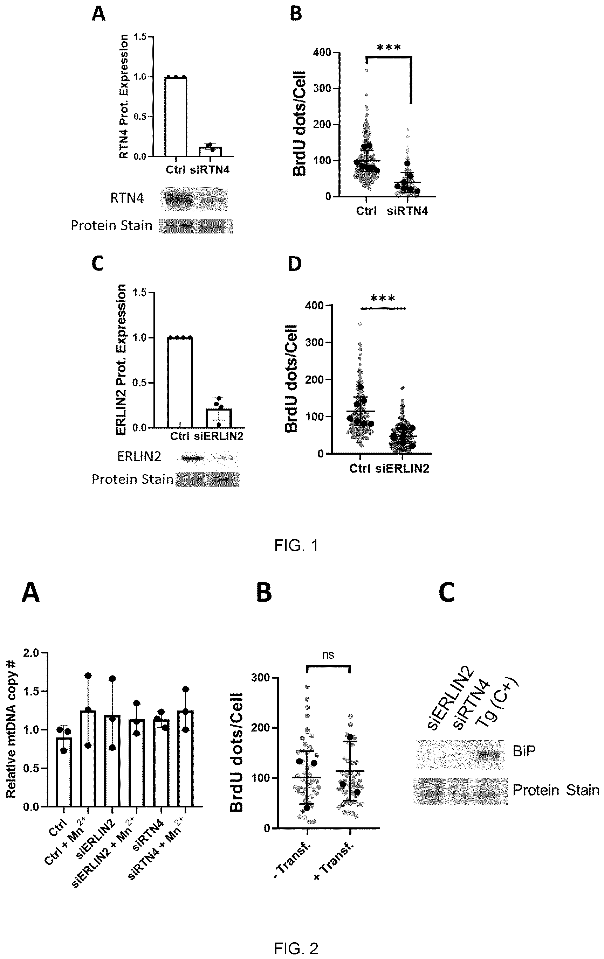

**Figure 1****.** Reticulon 4 and ER lipid raft protein 2 gene silencing inhibits mtDNA replication in primary human fibroblasts. Cells were transfected with and without verified silencer RNA^{Tm} targeting *RTN4* or *ERLIN2* (ThermoFisher) and decreased expression of the target protein validated via immunobloting panels **A** and **C,** respectively. **B**) Cells treated without and with silencer RNAs targeting *RTN4* (Thermofisher s32767 and s32768) were pulse-labeled with BrdU for 8 hours and stained with an anti-BrdU antibody. The chart indicates the quantification of the cytoplasmic BrdU foci. Mann-Whitney analysis of Control cells (n = 233 cells, 7 independent experiments) versus *RTN4* silenced cells (n = 175, from 7 independent experiments), ****p* < 0.001. **D**) Cells treated without and with silencer RNAs targeting *ERLIN2* (Thermofisher s22014 and s22015) pulse-labeled with BrdU, and stained with an anti-BrdU antibody. The chart indicates the quantification of the cytoplasmic BrdU foci. Mann-Whitney analysis of UT cells (n = 233 cells, 7 independent experiments) versus *ERLIN2* silenced cells (n = 160) (n = 7 independent experiments) ****p* < 0.001.
**Figure 2****.** The distribution and abundance of the mitochondria and ER cisternae are unaffected by ERLIN2 scarcity. **A)** qPCR estimated mtDNA copy number in human fibroblasts transfected without (Ctrl) or with siRNAs to *ERLIN2, RTN4, AFG3L2.* **B)** Cytoplasmic BrdU foci quantified in cells transfected with (+ Transf.) and without (-Transf.) a verified Scrambled siRNA. **C)** BiP, the canonical marker of the unfolded response is augmented in response to Thapsigargin (Tg) exposure, but not *RTN4* or *ERLIN2* silencing.

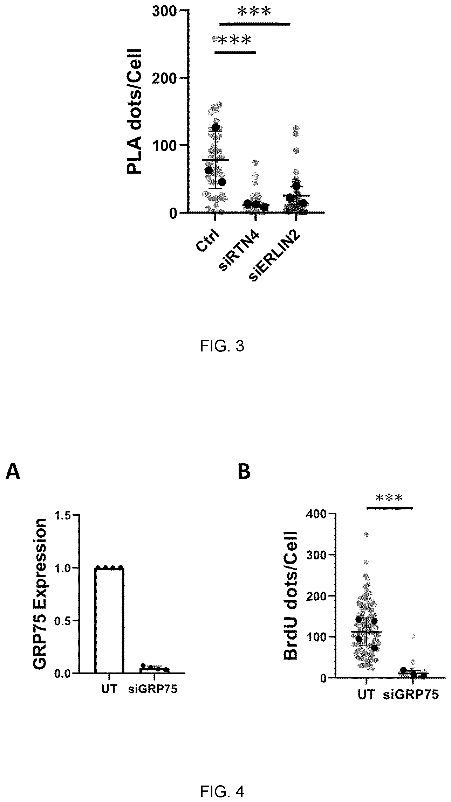

**Figure 3****.** *ERLIN2* silencing disrupts ER-mitochondrial junctions. IP3R1 and GRP75 antibodies were employed in proximity ligation assays on control and *RTN4* and *ERLIN2* silenced cells, and the GRP75/IP3R1 PLA signal quantified. Kruskal-Wallis post-hoc analysis of 54 Ctrl cells versus 91 *RTN4* silenced cells and 74 *ERLIN2* silenced cells (n = 3 independent experiments), ***p < 0.001.
**Figure 4****.** Gene silencing of *GRP75* inhibits mtDNA replication. After transfection of primary human fibroblasts with and without a verified silencer RNA targeting *GRP75* (Thermofisher s6989 or s6990), we evaluated: **A)** quantification of the steady state level of GRP75 via immunobloting after SDS-PAGE. **B)** BrdU labeling of the actively replicating DNA in the cytoplasm. Mann-Whitney analysis was applied in Control (Ctrl) cells (n = 113), and GRP75 silenced cells (n=52) in 3 independent experiments ***p < 0.001.

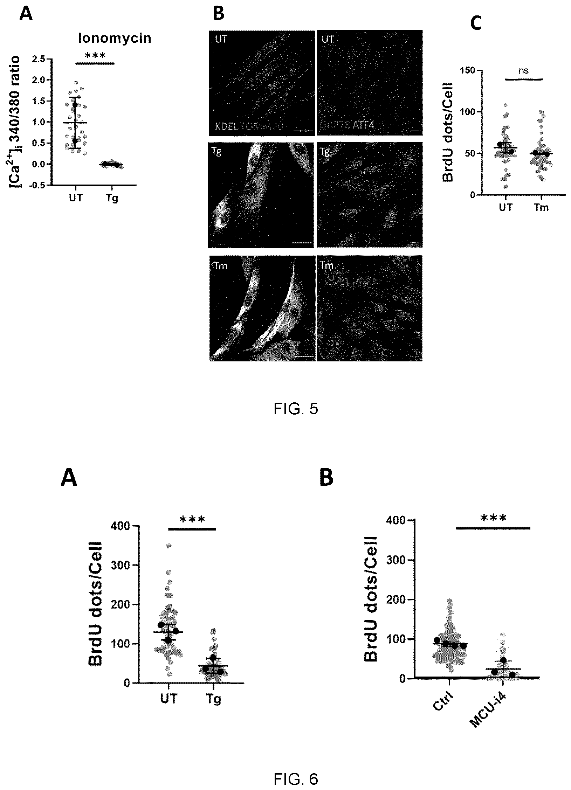

**Figure 5****.** Cellular effects of thapsigargin and tunicamycin. **A)** Change in cytosolic calcium signal after ionomycin treatment. Ionomycin induces almost no increase in cytosolic calcium in thapsigragin treated cells (100 nM Tg, 16 hours), concordant with the near complete absence of ER calcium stores owing to the inhibition of the SERCA pumps. Unpaired Student's t-test was applied to n = 30 untreated cells (UT), versus Thapsigargin-treated cells (n=32), ***p < 0.001 from 2 independent experiments. **B)** Both Thapsigargin and Tunicamycin (Tm) (1 µM, 16 hours) induce the UPR based on antibody detection of KDEL, GRP78/BiP and ATF4; however, **C)** Tm had no significant effect on mtDNA synthesis, in the presence of BrdU for the final 8 hours, in contrast to Tg (Fig. 6A). Mann-Whitney analysis was applied in untreated (UT) (n = 58), and Tunicamycin-treated cells (n=60) from 2 independent experiments; ***p < 0.001.
**Figure 6****.** Depletion of ER calcium stores and inhibition of MCU repress mitochondrial DNA replication. **A)** Fibroblasts were treated with 100 nM Tg for 16 hours, in the presence of BrdU for the final 8 hours and the cytoplasmic BrdU foci quantified in Tg-treated cells (n = 59) versus untreated (UT) cells (n = 49); Mann-Whitney analysis ****p* < 0.001 (n = 3 independent experiments). **B)** Cells treated without and with MCU-i4 and pulse-labeled with BrdU were stained with anti-BrdU antibody (green) and the cytoplasmic BrdU positive foci quantified. Mann-Whitney analysis of BrdU signal in control (Ctrl) cells versus MCU-i4 treated cells (146 and 96 cells, respectively, from 3 independent experiments), ****p* < 0.001.

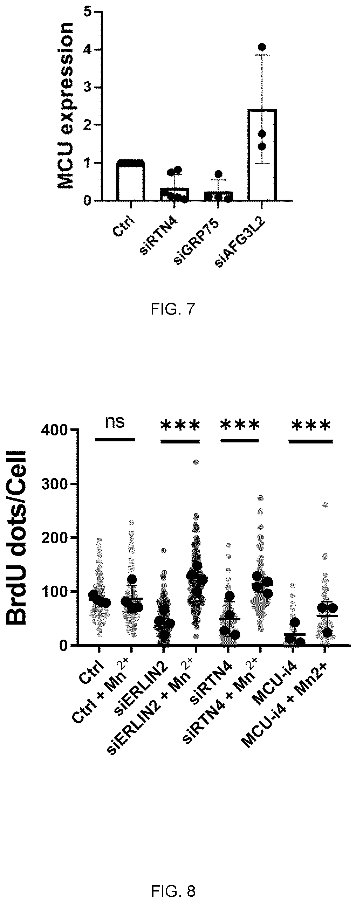

**Figure 7****. ERMCS disruption lowers MCU abundance,** Primary human fibroblasts were treated with or without siRNAs targeting *RTN4, GRP75 or AFG3L2* and extracted proteins analyzed by immunobloting of MCU and quantified.
**Figure 8****.** Manganese supplementation rescues mtDNA replication after *ERLIN2* or *RTN4* silencing, and inhibition of the mitochondrial calcium uniporter. Primary human fibroblasts were cultured with (+) or without (-) 50 µM Manganese (Mn²⁺) and labeled with BrdU, after *ERLIN2* or *RTN4* silencing or treatment with 10 µM MCU-i4 (MCU inhibitor). The chart represents the quantification of the cytoplasmic BrdU foci and Kruskal-Wallis post-hoc analysis of Ctrl (n= 144), siERLIN2 (n= 98), siRTN4 (n= 91), MCU-i4 (n= 97) Ctrl + Mn²⁺ (n= 153), siERLIN2 + Mn²⁺ (n= 137), siRTN4 + Mn²⁺ (n= 118), MCU-i4 + Mn²⁺ (n= 88) cells respectively (n = 3-4 independent experiments); ****p* < 0.001.

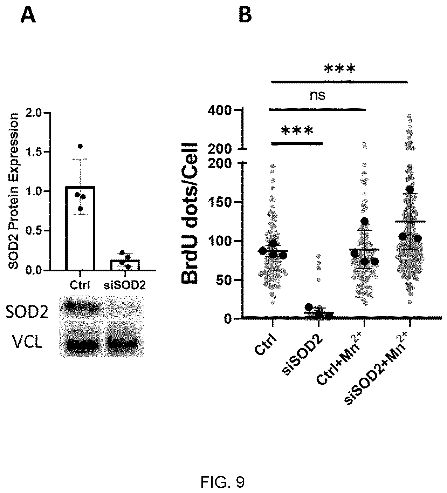

**Figure 9****.** Superoxide dismutase 2 (SOD2) gene silencing inhibits mtDNA replication, an inhibition that is rescued by manganese. **A)** Cells were transfected with and without a verified silencer RNA^{Tm} targeting *SOD2* (ThermoFisher) and decreased expression of the target protein validated via immunobloting. **B)** Primary human fibroblasts were cultured with or without 50 µM Manganese (Mn²⁺) and labeled with BrdU, after SOD2 silencing. Quantification of the cytoplasmic BrdU foci and Kruskal-Wallis post-hoc analysis of Ctrl (n= 144), siSOD2 (n= 89), Ctrl + Mn²⁺ (n= 153), siSOD2+ Mn²⁺ (n= 278) cells respectively (n = 3 independent experiments); ****p* < 0.001.

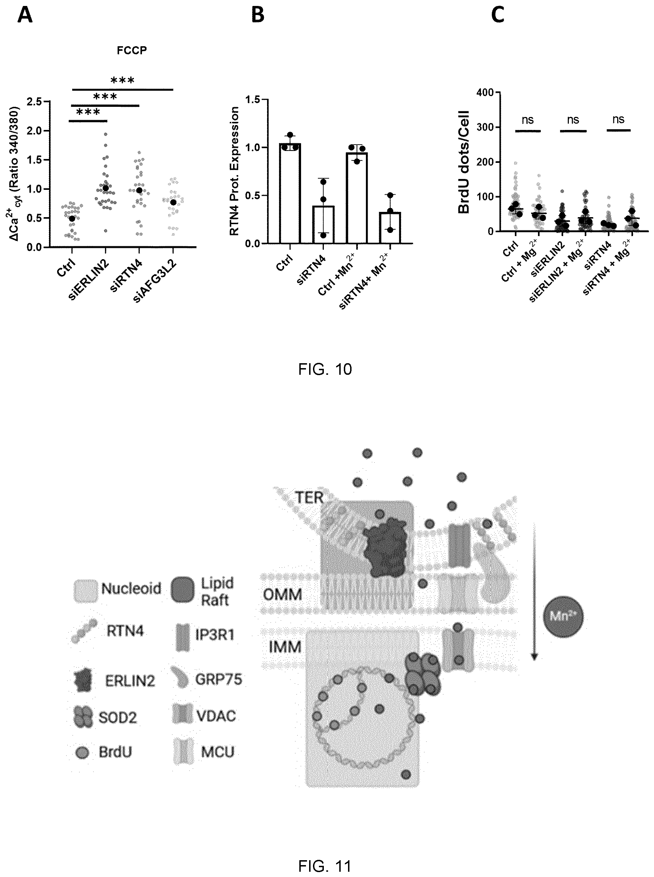

**Figure 10****.** The inhibition of mtDNA synthesis after ERMCS depletion is not owing to a shortage of calcium in the mitochondria, manganese does not interfere with *RTN4* silencing, and magnesium supplementation does not rescue mtDNA synthesis in *RTN4* or *ERLIN2* silenced cells. **A)** The increase in Fura2M-detected cytosolic calcium, ΔCa²⁺, after inducing the release of mitochondrial calcium with the uncoupler FCCP, was analyzed in control Ctrl, *ERLIN2, RTN4* and *AFG3L2* silenced cells. **B)** Primary human fibroblasts were cultured with (+) or without (-) 50 µM Manganese (Mn²⁺) after *RTN4* silencing and extracted proteins analyzed by immunobloting of RTN4 whose abundance was normalized with respect to vinculin (VCL). **C)** Primary human fibroblasts were cultured with (+) and without (-) 50 µM Magnesium (Mg²⁺) and labeled with BrdU for 14 hours, after which, incorporated BrdU was immunodetected and the cytoplasmic foci quantified and subjected to a Kruskal-Wallis post-hoc analysis of Ctrl (n= 80), Ctrl + Mg²⁺ (n= 45), siERLIN2 (n= 67), siERLIN2 + Mg²⁺ (n= 46), siRTN4 (n= 70), siRTN4 + Mg²⁺ (n= 64) cells, respectively (n = 3 independent experiments); ****p* < 0.001.
**Figure 11****.** ERMCS Regulate Mitochondrial DNA Replication via Manganese. Reticulon 4 (RTN4) is a component of tubular ER (TER) which is required to form connections with the mitochondria (ERMCS), and RTN4 expression is proportional to mtDNA synthesis (Figure 1 and Lewis et al. 2016). ERLIN2 is a fundamental component of ER lipid rafts, without which ERMCS cannot form (Figure 3), and so it too supports mtDNA synthesis (Figure 1). Equally, because GRP75 is a bridging component of ERMCS (Szabadkai et al., 2006), its repression inhibits mtDNA replication (Figure 4). The abundance of the mitochondrial calcium uniporter, MCU, correlates with that of RTN4, ERLIN2 and GRP75 (Figures 4A and 4B). Hence, mtDNA synthesis is proportional to ERMCS and MCU abundance (Figures 7 and 11). Oppositely, repression of the m-AAA protease AFG3L2 enhances mtDNA replication correlated with increased MCU and ERMCS (Figure 7). Although calcium transfer is recognized as a major function of ER-mitochondrial connections, mitochondrial calcium stores are not depleted by ERMCS scarcity, but another metal cation substrate of MCU, manganese, restores mtDNA synthesis after silencing of ERMCS factors (Figure 8). IMM and OMM - inner and outer mitochondrial membranes, respectively; VDAC - voltage dependent anion channel, aka porin.

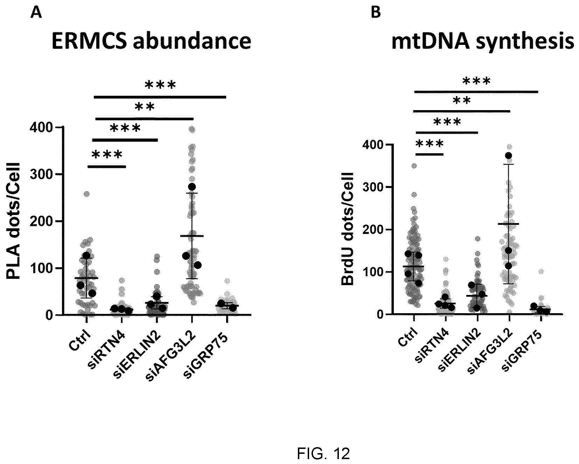

**Figure 12****.** ERMC abundance is directly proportional to mtDNA replication. The charts conflate the PLA data (A) and the BrdU data (B) that appear in several of the other figures to highlight the consistent correlation between ERMC numbers and mtDNA synthesis.

### Examples

### I. MATERIAL AND METHODS

**Chemicals and cell culture.** All chemicals, unless otherwise stated, were purchased from Sigma Aldrich with respective CAS number: Tg (67526-95-8), Tm (11089-65-9), ATP (34369-07-8), BrdU (59-14-3), MCU-i4 (371924-24-2), MnCl₂ (13446-34-9), Cyclohexamide (Cas number 66-81-9), Actinonin (Cas number 13434-13-4), HPG Click.it ^{®} ThermoFisher (C10186). Primary human fibroblasts were routinely maintained in Dulbecco's Modified Eagle's Medium (DMEM) (Gibco) containing 25 mM glucose, 1 mM pyruvate supplemented with 10% fetal bovine serum (FBS, Gibco), 5% penicillin and streptomycin (Gibco), 5% CO₂, at 37°C. All the cell lines were regularly confirmed free of mycoplasma, using the Venor Gem Classic Mycoplasma PCR Detection Kit (Minerva Biolabs).

**Gene silencing.** For RNAi experiments, primary human fibroblasts were transfected twice (the second one, 72 hours after the first) with Lipofectamine^{™} RNAiMAX (Invitrogen) and 10 nM double-stranded (ds) RNA targeting gene of interest (Table 1). (ThermoFisher Silencer Pre-designed siRNAs. Analysis of proteins and nucleic acids *in situ*, or on extracted material was performed 144 hours after the first transfection.

**Table 1. Identity and source of the Silencer RNAs.**

| **Silencer select** | **Manufacturer** | **Catalogue #** | **siRNA IDs** |
|---|---|---|---|
| RTN4 | ThermoFisher | 4392420 | S32767, s32768 |
| ERLIN2 | ThermoFisher | 4427037 | S22014, s22015 |
| AFG3L2 | ThermoFisher | 4427038 | S21516, s21517 |
| GRP75 | ThermoFisher | 4427037 | S6989, s6990 |
| SOD2 | ThermoFisher | 4427038 | s13267, s13269 |
| Scrambled | Qiagen | 1022076 | 1022076 |

**Immunofluorescence.** Fibroblasts were grown on 96-well black microplates (Ibidi),and fixed with 4% paraformaldehyde (Electron Microscopy Sciences) for 20 minutes at room temperature (RT). For the bromo-deoxyuridine (BrdU, Sigma) incorporation experiments, cells were incubated with BrdU 50 µM for 8 hours before fixation. After washing with Phosphate Buffered Saline (DPBS, Gibco), the cells were permeabilized with 0.3% Triton X-100 (Santa Cruz Biotechnologies) for 30 minutes in PBS and treated with 2N HCl for 30 minutes at 37°C. After five washes for 10 minutes with PBS, cells were blocked for 1 hour at RT with 10% goat serum (Sigma Aldrich) in 0.1% Triton X-100, PBS (PBST). Cells were then incubated with primary antibodies (Table 2) in PBST overnight (O/N) at 4°C. After three washes for 5 minutes with PBST, cells were incubated with the appropriate secondary antibodies (Table 2) for 1-2 hours at RT. Finally, cells were washed with PBS and covered with mounting Medium containing 4',6-diamidino-2-phenylindole (DAPI) nuclear stain (ibidi). For image staining other than BrdU, the same protocol was followed omitting the acid treatment.

**Table 2. Identity, source and dilutions of the primary and secondary antibodies used in the study.**

| **Primary Antibody** | **Manufacturer** | **Catalogue #** | **Dilution for ICC** (for WB is 1:1000) |
|---|---|---|---|
| Anti-DNA (mouse) | Progen | 690014S | 1:250 |
| BrdU (rat) | Abcam | Ab6326 | 1:250 |
| TOMM20 (rabbit) | Proteintech | 11802-1-AP | 1:250 |
| TOMM20 (mouse) | Santa Cruz Biotechnology | Sc-17764 | 1:250 |
| GRP75 (mouse) | Santa cruz Biotechnology | Sc-133137 | 1:200 |
| IP3R1 (rabbit) | Novus | NB120-5908 | 1:200 |
| ERLIN2 (rabbit) | Sigma Aldrich | HPA002025 | 1:200 |
| NOGO (mouse) | Santa Cruz | Sc-271878 | Only WB |
| RTN4 (rabbit) | Atlas | HPA023977 | Only ICC 1:250 |
| AFG3L2 (rabbit) | Proteintech | 14631-1-AP | 1:250 |
| KDEL (mouse) | Novus | NBP1-97469 | 1:700 |
| GRP78/BiP (rabbit) | Abcam | ab21685 | 1:1000 |

| **Secondary Antibody for ICC** | **Manufacturer** | **Catalogue #** | **Dilution for ICC** |
|---|---|---|---|
| IgG (H+L) Highly Cross- Adsorbed Goat anti-Rat,Alexa Fluor^{™} 488 | Invitrogen | A-11006 | 1:500 |
| IgG (H+L) Cross-Adsorbed Goat anti-Rat, Alexa Fluor^{™} 555 | Invitrogen | A23434 | 1:500 |
| Alexa Fluor^{®}-488 goat-anti-mouse IgG | Invitrogen | A11001 | 1:500 |
| Alexa Fluor^{®}-555 goat-anti-mouse IgG | Invitrogen | A21422 | 1:500 |
| Alexa Fluor^{®}-555 goat-anti-rabbit IgG | Invitrogen | A21428 | 1:500 |
| Alexa Fluor^{®}-488 goat-anti-rabbit IgG | Invitrogen | A-11008 | 1:500 |
| Anti-mouse MINUS | Sigma Aldrich | DUO92004 | 1:5 |
| Anti-rabbit PLUS | Sigma Aldrich | DUO92002 | 1:5 |

| **Secondary Antibody for WB** | **Manufacturer** | **Catalogue #** | **Dilution for WB** |
|---|---|---|---|
| Goat Anti-MouseIgG (H+L), HRP | Promega | W4021 | 1:5000 |
| Goat Anti-RabbitlgG (H+L), HRP | Promega | W4011 | 1:5000 |
| Goat Anti-rabbit IgG, AlexaFluor-647 PLUS | Invitrogen | A32733 | 1:5000 |
| Goat Anti-mouse IgG, AlexaFluor-647 PLUS | Invitrogen | A32728 | 1:5000 |

Proximity ligation assay (PLA) staining was carried out following the manual of the commercial house Merck. Cells were permeabilized with 0.3% Triton X-100 (Santa Cruz Biotechnologies) for 30 minutes in PBS. Cells were incubated with blocking solution in a heated humidity chamber for 60 minutes at 37°C. Cells were then incubated with primary antibodies to GRP75 (Santa Cruz Biotechnologies) and IP3R1 (NOVUS) (Table 2) in Antibody Diluent overnight at 4°C. After two washes for 5 minutes with wash buffer A (WB-A), cells were incubated with anti-mouse MINUS (DUO92004) and anti-rabbit PLUS (DUO92002) (Table 2) in antibody diluent, in a pre-heated humidity chamber for 1 hour at 37°C. After two washes for 5 minutes with WB-A, cells were incubated in a pre-heated humidity chamber for 30 minutes at 37°C for the ligation step. After two 5 minutes washes with WB-A, cells were placed in a pre-heated humidity chamber for 100 minutes at 37°C with the secondary antibody (Table 2) for the amplification step. Finally, cells were washed twice for 10 minutes with WB-B followed by 0.01X WB-B for 1 minute, and covered with mounting Medium containing 4',6-diamidino-2-phenylindole (DAPI) nuclear stain.

**Fractionation and immunodetection of proteins.** Cells were trypsinized and lysed on ice with RIPA buffer (150 mM NaCl, 1.0% TritonX-100, 0.5% sodium deoxycholate, 0.1% SDS, 50 mM Tris, pH 8.0) 1x Halt^{™} Protease and Phosphatase Inhibitor Cocktail (Thermo Scientific). After incubating on ice for 40 minutes, the samples were centrifuged for 20 minutes at 15,000 g, 4°C to remove cellular debris. Protein concentration was determined by DC protein assay kit (Biorad). Protein samples were prepared in 1x Laemmli loading buffer (Biorad) with DTT and resolved on 4-15% 4-20% or 12% Mini-PROTEAN^{®} TGX^{™} Precast Gels (Biorad) run in Tris/Glycine/SDS running buffer (Biorad). After electrophoresis, proteins were transferred overnight to Low-Fluorescence PVDF Transfer Membranes (Thermo Scientific) at 20 V, at 4°C. Membrane was washed with PBS for 5 minutes and incubated with No-Stain^{™} Protein Labeling Reagent (Thermo Fisher) for 15 minutes followed by iBright FL1500 Imaging System visualization. After washing the membrane with PBS for 2 minutes, membrane was blocked in 3% BSA in PBS, 0.1% Tween for 1 hour at RT. Membranes were incubated O/N with primary antibodies (see Table 2) in BSA 3% in PBS, 0.1% Tween, at 4°C and, after washing, incubated with the appropriate secondary antibody (see Table 2) for 1 hour at RT. Proteins were detected using standard ECLTM Western Blotting Analysis System (GE Healthcare) or SuperSignal^{®}West Dura (Thermo Scientific), and acquired and quantified using an iBright FL1500 Imaging System.

**Quantification of mtDNA.** The mtDNA copy number was quantified as follows: after DNA isolation, real-time quantitative PCR was performed in triplicates on 384-Well Reaction Plates (Applied Biosystems) in final volumes of 13 µL. Each reaction contained 15 ng of DNA template, 1x Power SYBRGreen PCR Master Mix (Applied Biosystems) and 0.5 µM of forward and reverse primers. Mitochondrial and nuclear DNA were amplified using primers specific to regions of human t-RNA and B2 microglobulin genes respectively. t-RNA forward sequence (5'-CACCCAAGAACAGGGTTTGT-3' SEQ ID NO: 1 tRNALeuUURF. 2) and reverse sequence (5'- TGGCCATGGGTATGTTGTTA-3' SEQ ID NO: 2 tRNALeuUURR. 3). B2 microglobulin forward sequence (5'-TGCTGTCTCCAT GTTTGATGTATCT-3' SEQ ID NO: 3 BMGF. 4) and reverse sequence (5'-TCTCTGCTCCCCACCTCTAAGT-3' SEQ ID NO: 4 BMGR. 5). Changes in the mtDNA copy number were determined by using the 2-ΔΔCt method (Schmittgen, T. D. & Livak, K. J. (2008). Nat Protoc 3, 1101-1108) and represented as fold-change relative to the mean value for vehicle-treated cells analyzed in parallel (Dalla Rosa, I. et al., (2016). PLoS genetics, 12(1), e1005779).

**Image capture and analysis.** Fluorescent labeling images were captured by a Nikon Eclipse 80i epifluorescence microscope or LSM 900 KMAT with AXIO OBSERVER 7 Zeiss confocal microscope (Nikon), using the microscope software (NIS Elements Software) in an ".nd2" format. Laser power, gain and offset parameters were kept constant for each experiment. The image analysis was performed using the plugins available in Fiji imaged (2.0.0-rc-15/1.49h); any adjustments to brightness and contrast were applied linearly to all images in a comparison.

**Calcium measurements.** Cytosolic calcium imaging was analyzed using the ratiometric calcium dye Fura-2AM (ThermoFisher F1201). Cells were loaded with 4 µM Fura-2AM in the presence of 0.02% pluronic acid for 30 minutes at 37°C in culture medium. Cells were washed with Ringer solution (125 mM NaCl, 5 mM KCI, 1.2 mM MgSO₄, 6 mM glucose, 2 mM CaCl₂ and 25 mM HEPES, pH 7.4) and after 15 minutes, images were acquired with an ECLIPSE Ti/L100 microscope (Nikon) equipped with a 20X S-Fluor objective, a lambda-DG4 illumination system, and an Orca-Flash 2.8 camera (Hamamatsu). NisElements-AR software was used for data analysis. Intracellular calcium concentration was estimated by the ratio of Fura-2AM fluorescence intensities at excitation wavelengths 340 nm and 380 nm, using in situ calibration, as previously described (Lasa-Elgarresta, J., et al. (2022). Front Cell Dev Biol 10, 822563). Organelle calcium content measurements were as follows. In a calcium free medium, 1 µM ionomycin after 1 µM FCCP, was used to estimate ER calcium content. Alternatively, in a calcium free medium, 100 µM ATP was added to cells to activate P2Y receptors and thereby induce Ca²⁺ release from the ER via IP3 receptors (Mencacci et al., (2015). Am J Hum Genet, 96, 938-947). Thapsigargin is an inhibitor of the ER calcium ATPase (SERCA) that prevents calcium uptake from the cytosol, allowing an estimation of the ER Ca²⁺ pool (Lytton, et al., (1991). J Biol Chem 266, 17067-17071).

**Data Analysis and Statistical Procedures.** GraphPad Prism version 8.01 was used to perform statistical analyses. Data distribution was evaluated with Shapiro-Wilk and D'Agostino & Pearson omnibus normality tests. Data having a Gaussian distribution were analyzed using unpaired Student's t-test. When data did not follow a normal distribution, Mann-Whitney test were used. When comparing more than two groups, One-Way ANOVA followed by Kruskal-Wallis post-hoc test was applied. For all statistical analyses, the adjusted p-values of less than 0.05 were considered statistically significant, **p* < 0.05, ***p <* 0.01, ****p <* 0.001, respectively.

### II. RESULTS

### The ER protein ERLIN2 supports mitochondrial DNA replication

The seminal study that indicated that ER connections with mitochondria play a role in mtDNA replication and segregation assigned a critical role in the process to tubular ER by showing that ER tubule versus ER cisternae abundance was proportional to the rate of mtDNA synthesis. Here, applying another genetic manipulation, we silenced the critical ER tubular factor *RTN4,* in primary human fibroblasts with short interfering RNAs (siRNA), and measured the newly synthesized mtDNA via bromodeoxyuridine (BrdU) incorporation. One round of transfection had only a modest effect on the steady-state level of Reticulon 4 protein, but two rounds produced an 89% decreased compared to controls (Fig. 1A), and mtDNA synthesis fell by 65% (Fig. 1B), indicating that the tubular ER supports mtDNA replication in primary cultured human cells, as well as in osteosarcoma cells.

As ERLIN2 is a component of ER cholesterol microdomains and a canonical marker of the ERMCS, we next assessed whether ERLIN2 is functionally important for mtDNA synthesis. *ERLIN2* gene silencing decreased the expression of the target protein by 80% (Fig. 1C), and the incorporation of BrdU into cytoplasmic puncta (mtDNAs) decreased by 55%, (Fig. 1D). Neither *RTN4* nor *ERLIN2* silencing altered the mtDNA copy number (Fig. 2A) or its distribution; hence, we infer that the decrease in BrdU incorporation is indicative of a marked inhibition of mtDNA synthesis owing to ERMCS depletion (and later confirmed by PLA analysis, see below and Fig. 3). No such decrease in mtDNA synthesis was evident when cells were transfected with scrambled RNAs, which excluded an indirect effect of transfection of exogenous RNA (Fig. 2B). And although many stresses can induce the unfolded protein response (UPR) or cause mitochondrial fragmentation, ERLIN2 repression did not induce the UPR (Fig. 2C), alter the ER cisternae, or disrupt the mitochondrial network.

### ERLIN2 maintains ER-mitochondrial junctions

Because *ERLIN2* silencing markedly inhibits the mtDNA synthesis (Fig. 1D), we hypothesized that ER-mitochondrial connections are dysfunctional or lacking when ERLIN2 is scarce. To determine the overall contribution of ERLIN2 to ERMCS, we performed proximity ligation assays (PLA) with antibodies to two components of the junctions, inositol 1,4,5-trisphosphate receptor type1 (IP3R1) and glucose regulated protein 75 (GRP75), localized in the ER and mitochondria, respectively. In control cells, PLA produced many cytoplasmic foci, consistent with IP3R1 and GRP75 being juxtaposed ERMCS. *RTN4* silencing diminished the GRP75/IP3R1 PLA signal by 90%, as expected given that RTN4 is required for tubular ER that supports ERMCS formation (Fig. 3). *ERLIN2* silencing produced a similar marked decrease in the PLA signal (Fig. 3). These results indicate ERLIN2 is a critical contributor to ER-mitochondrial junctions, which explains the decrease in mtDNA synthesis associated with its repression (Fig. 1D). Seeking further confirmation that ERMCs are fundamental to mtDNA synthesis in human fibroblasts, we repressed another core component of the junctions, GRP75, via RNA interference, and found that this decreased BrdU incorporation in newly synthesized mtDNA, as well as decreasing ERMCs, similar to the repression of *ERLIN2* and *RTN4* (Fig. 4).

### Depletion of ER ion stores inhibits mtDNA replication

Calcium transfer occurs via ERMCs, and calcium is known to stimulate various aspects of mitochondrial function, such as the tricarboxylic acid cycle. Therefore, we investigated the depletion of ER calcium affects mtDNA replication, using Thapsigargin (Tg), which irreversibly inhibits the SERCA pumps that transport cations into the ER. Human fibroblasts were treated with and without 100 nM Tg for 8 or 16 hours, in the presence of BrdU for the final 8 hours. Thapsigargin decreased the ER calcium load as expected (Fig. 5A) and this was accompanied by an 88% decrease in mtDNA synthesis, as measured by BrdU incorporation in cytoplasmic foci within the mitochondrial network, compared to untreated cells (Fig. 6A). Although Thapsigargin induces the Unfolded Protein Response (Fig. 5B), the canonical activator of the UPR tunicamycin (Tm) did not appreciably alter mtDNA synthesis (Fig 5C), and so ER-stress is not responsible for Thapsigargin's effect on mtDNA synthesis.

### Inhibition of MCU Impedes Mitochondrial DNA Synthesis

The mitochondrial calcium uniporter, MCU, plays a major role in mitochondrial calcium homeostasis and so was the prime candidate for regulating entry of calcium, or a related cation, into the mitochondria from the ER. With the hypothesis that MCU is the conduit for the ER factor that stimulates mtDNA replication, we applied the MCU inhibitor, MCU-i4 (10 µM) (Di Marco G, 2020) and found it markedly decreased nascent mtDNA synthesis in fibroblasts, similar to ERMCS depletion (Fig. 6B vs. Fig. 1). Moreover, *RTN4* and *GRP75* silencing, which decrease ERMCS and inhibit mtDNA synthesis (Figs. 1 and Fig. 4B), were accompanied by decreases of 65% and 75% in the steady-state level of MCU protein, respectively (Fig. 7). On the other hand, silencing the m-AAA Protease AFG3L2, which has been shown to limit the activity of MCU in neuronal mitochondria, did not decrease MCU abundance or mtDNA synthesis (Fig. 7).

Although the strong correlation between ERMCS, MCU and mtDNA synthesis suggested calcium flux from the ER to the mitochondria might support mtDNA synthesis, calcium persists in mitochondria in the absence of MCU *in vivo,* and mitochondrial calcium stores are not depleted by *RTN4* or *ERLIN2* silencing, evidenced by the amount of calcium released to the cytosol following mitochondrial depolarization (Fig. 10A). Therefore, we next considered another metal cation that is transported into mitochondria via MCU, manganese. To determine whether manganese scarcity might be responsible for the decrease in mtDNA synthesis in the absence of ERMCS, we again repressed *RTN4* and *ERLIN2* and supplemented the cells with and without 50 µM manganese chloride. Manganese supplementation did not interfere with the gene silencing, as it had no effect on RTN4 abundance (Fig. 10B), nor did it have an appreciable effect on mtDNA synthesis in control cells; however, it increased mtDNA synthesis in *RTN4* and *ERLIN2* silenced cells 6-fold and 5-fold, respectively, exceeding that of control cells (Fig. 8). In contrast to manganese, additional magnesium (Mg²⁺) had no impact on the repressed mtDNA synthesis (Fig. 10C). Manganese (Mn²⁺) was able to augment mtDNA synthesis 3.6-fold in cells treated with the MCU inhibitor MCU-i4 (Fig. 8). Given that manganese can restore mtDNA synthesis when ERMCS are scarce or MCU is inhibited, we infer manganese is the ER factor that ordinarily transfers from the ER to mitochondria to promote mtDNA replication and that it crosses the inner ion-impermeable mitochondrial membrane via MCU. Presumably mitochondrial transporters other than MCU have a low affinity for manganese enabling enough of it to enter the organelles to support mtDNA synthesis when the metal cation is present at high levels, whereas at normal cellular levels manganese import into mitochondria depends on MCU.

### Manganese-dependent superoxide dismutase, SOD2, regulates mitochondrial DNA replication

The next question arising was the fate of manganese on entering the mitochondrial matrix. Manganese is an essential co-factor of superoxide dismutase 2, SOD2 that has been found to be associated with mitochondrial nucleoprotein complexes (nucleoids) and has been linked to the activities of mitochondrial DNA polymerase (POLG), although hitherto this was assumed to reflect a protective role against mtDNA damage. To determine whether the striking effect of manganese on mtDNA synthesis involved SOD2, we measured BrdU incorporation in the mtDNA after silencing SOD2 and found that the reduced level of SOD2 decreased mtDNA synthesis 93%, an effect even greater than silencing ERMCS components (Fig. 9 vs Fig. 1). Moreover, when SOD2 was scarce, manganese supplementation fully restored mtDNA synthesis: 130% of the level of untreated controls, a 20-fold increase with respect to SOD2 silenced cells without manganese (Fig. 9). These findings suggest that SOD2 utilizes manganese to stimulate mtDNA synthesis; however, SOD2 appears to be a conduit, as in its absence, high levels of manganese can directly enhance mtDNA synthesis.

### Model for ERMCS support of mtDNA replication

The current study provides a model of how the ER supports the replication of mtDNA and identifies key regulatory and structural elements (Fig. 11). In addition to RTN4, the ER lipid raft protein ERLIN2, and the mitochondrial ERMC bridging protein GRP75 contribute to mtDNA replication, and this reflects the fact that ERMC abundance is directly proportional to mtDNA replication (Fig. 12). On the mitochondrial side of the junctions, MCU and SOD2 propagate the ER signal, because inhibiting the former and repressing the latter reduces mtDNA synthesis similar to depleting ERMCS (Fig. 8 and Fig. 9).

All the evidence points to manganese as the specific signaling factor, as it is an established substrate of MCU, the essential co-factor of SOD2 (aka manganese-dependent superoxide dismutase), and most compellingly manganese supplementation rescues mtDNA synthesis when ERMCS and SOD2 are scarce, or MCU is inhibited (Fig. 8, Fig. 9).

Given that ERMCS can profoundly impact mtDNA replication, mtDNA metabolism merits investigation in all the contexts where aberrant ER-mitochondrial connections have been linked to disease, such as neurodegenerative disorders. More specifically, mutations in ERLIN2 and AFG3L2 are established causes of motor neuron disease, and so dissection of mtDNA metabolism could provide important insights into the pathogenesis of these disorders. Another important corollary is that one or more of the widely reported consequences of calcium perturbation in neurological and neurodegenerative disorders could instead stem from the accompanying changes in manganese availability and the consequent adverse effects on mtDNA replication. Equally, altered MCU and SOD2 abundance and activity needs to consider the impact on mtDNA replication. MCU forms a multi-protein complex comprising ERME, MICU1 and MICU2, as well as MCU itself; hence, these partners may well play important roles in modulating the activity of the transporter, as it relates to manganese entry into mitochondria and thus mtDNA replication.

## Claims

1. A composition comprising manganese, a solvate or a pharmaceutically acceptable salt thereof, for use in the treatment of a disease in a subject, wherein the subject's cells have fewer than half the number of functional endoplasmic reticulum and mitochondria contact sites (ERMCS) than the cells from a subject not suffering from the disease.

2. Composition according to claim 1, wherein the disease is a neurodegenerative disease.

3. Composition for use according to claim 2, wherein the neurodegenerative disease is selected from Alzheimer's disease, Parkinson's disease, Diffuse Lewy Body Disease (DLBD), Huntington's disease, multiple sclerosis, or Amyotrophic lateral sclerosis (ALS) dementia.

4. Composition for use according to any one of claims 1 to 3, wherein the composition is comprised within a nanoparticle.

5. Composition for use according to any one of claims 1 to 4, wherein the amount of functional endoplasmic reticulum and mitochondria connections (ERMCS) is measured by a mtDNA synthesis assay.

6. An in vitro method for designing a therapy to a subject suffering from a disease, the method comprising
(a) determining the mtDNA synthesis rate in a biological sample isolate from the subject in the presence and absence of Mn, and
(b) comparing the mtDNA synthesis rates obtained in step (a),
wherein
- The mtDNA synthesis rate is measure by a mtDNA synthesis assay, and
- A 2-fold or greater increase in mtDNA synthesis rate in the presence of Mn with respect to the mtDNA synthesis rate in the absence of Mn, is indicative that the therapy to be administered to the subject is a composition comprising manganese, a solvate or a pharmaceutically acceptable salt thereof.

7. Method according to claim 6, wherein the dose of Mn to be administered to the subject will be proportional to the increase of the mtDNA synthesis rate in the presence of Mn with respect to the mtDNA synthesis rate without Mn.

8. Method according to claims 6 and 7, wherein the disease is a neurodegenerative disease.

9. Method according to claim 8, wherein the neurodegenerative disease is selected from Alzheimer's disease, Parkinson's disease, Diffuse Lewy Body Disease (DLBD), Huntington's disease, multiple sclerosis, or Amyotrophic lateral sclerosis (ALS) dementia.

10. Method according to any one of claims 6 to 9, wherein the composition is comprised within a nanoparticle.
